# EUROPEAN PATENT SPECIFICATION

(11) **EP 3 579 829 B1**
(45) Date of publication and mention of the grant of the patent: **25.03.2026**
(21) Application number: 18703972.2
(22) Date of filing: 06.02.2018
(51) Int. Cl.: A61K 31/202, A61K 9/00, A61K 9/107, A61P 1/16

(54) **LONG-TERM EFFICACY OF LIVER DISEASE TREATMENT WITH EPA AND DHA**
LANGZEITWIRKUNG VON LEBERERKRANKUNGSBEHANDLUNG MIT EPA UND DHA
EFFICACITÉ À LONG TERME DU TRAITEMENT D'UNE MALADIE DU FOIE PAR EPA ET DHA

(30) Priority: 07.02.2017 EP 17155032
(43) Date of publication of application: 18.12.2019
(73) Proprietor: Fresenius Kabi Deutschland GmbH, 61352 Bad Homburg (DE)
(72) Inventor: WESTPHAL, Martin, 61352 Bad Homburg (DE); BAASNER, Silke, 61352 Bad Homburg (DE); HONNDORF, Stefanie, 61352 Bad Homburg (DE); BRITO DE LA FUENTE, Edmundo, 61348 Bad Homburg (DE); GALLEGOS-MONTES, Crispulo, 61352 Bad Homburg (DE); QUINCHIA-BUSTAMANTE, Lida, 61169 Friedberg (DE)
(74) Representative: Fresenius Kabi Deutschland GmbH
(86) International application number: PCT/EP2018/052873
(87) International publication number: WO 2018/146060

(56) References cited:
- WO-A1-2015/053379
- WO-A1-2016/057915
- US-A1- 2013 156 861
- S. CAZANAVE ET AL: "Omega-3 Carboxylic Acids, Epanova , and the Sodium-Glucose Co-Transporter 2 Inhibitor, Dapagliflozin , Improve Steatohepatitis and Fibrosis Scoring in a Mouse Model of Non-Alcoholic Steatohepatitis", JOURNAL OF HEPATOLOGY, vol. 64, no. 2, 2016, AMSTERDAM, NL, pages S685, XP055393037, ISSN: 0168-8278, DOI: 10.1016/S0168-8278(16)01303-9
- BRIAN T. KALISH ET AL: "A Metabolomic Analysis of Two Intravenous Lipid Emulsions in a Murine Model", PLOS ONE, vol. 8, no. 4, 2 April 2013 (2013-04-02), pages e59653, XP055392632, DOI: 10.1371/journal.pone.0059653
- MATTHEW K ITO: "A Comparative Overview of Prescription Omega-3 Fatty Acid Products", P.T., vol. 40, no. 12, December 2015 (2015-12-01), pages 826 - 836, 857, XP055393029
- ELEONORA SCORLETTI ET AL: "Effects of purified eicosapentaenoic and docosahexaenoic acids in nonalcoholic fatty liver disease: Results from the WELCOME* study", HEPATOLOGY, vol. 60, no. 4, 25 August 2014 (2014-08-25), pages 1211 - 1221, XP055393112, ISSN: 0270-9139, DOI: 10.1002/hep.27289
- TANAKA NAOKI ET AL: "Highly purified eicosapentaenoic acid treatment improves nonalcoholic steatohepatitis", JOURNAL OF CLINICAL GASTROENTEROL, RAVEN PRESS LTD., NEW YORK, NY, US, vol. 42, no. 4, April 2008 (2008-04-01), pages 413 - 418, XP009167353, ISSN: 0192-0790, DOI: 10.1097/MCG.0B013E31815591AA

## Description

The present invention relates to the field of medical treatments of liver diseases. In particular, it relates to a composition for use in treating and/or preventing a liver disease in a subject, wherein said composition comprises eicosapentaenoic acid (EPA) and docosahexaenoic acid (DHA), and wherein the ratio of EPA to DHA is at least 2:1.

Fatty liver is a reversible condition wherein large vacuoles of triglyceride fat accumulate in liver cells via the process of steatosis (i.e., abnormal retention of lipids within a cell). Despite having multiple causes, fatty liver can be considered a single disease that occurs worldwide in those with excessive alcohol intake and the obese. Fatty liver disease is the most common liver disorder in developed countries.

Fatty liver disease includes a wide spectrum of disease stages, ranging from steatosis, which is characterized by at least 5% triglyceride accumulation, to nonalcoholic or alcoholic steatohepatitis (NASH/ASH), which is characterized by liver fat accumulation and inflammation. Fibrosis is seen in 20% of NASH/ASH patients, and hepatocellular carcinoma (HCC) develops in NASH/ASH livers even without cirrhosis. It is difficult to distinguish alcoholic fatty liver disease from nonalcoholic fatty liver disease, and both show microvesicular and macrovesicular fatty changes at different stages. Current treatment options for fatty liver diseases are limited and ineffective.

There is evidence for beneficial effects of omega-3 fatty acids in preclinical models for liver disease including hepatic steatosis, NASH and HCC. It was shown that treatment with Omegaven^{®} 10%, which is an emulsion for intravenous use containing the omega-3-fatty acids EPA and DHA in a ratio of approximately 1:1 in an amount of 2.4 g fat/kg body weight/day lead to an improved lipid metabolism as well as the reduction of oxidative stress in a model of hepatic steatosis (Kalish et al., 2013). Calculated to 75 kg bodyweight, this would result in an intake of 1620 kcal/day only through fat (fish oil).

The oral supplementation of the omega-3-fatty acids alpha-linolenic acid (ALA, 64%), EPA (16%) and DHA (21%) was associated with an improved liver histology in patients with biopsy-proven NASH after 3-6 months (Nogueira et al., 2015). Li et al., 2015 reported that the uptake of DHA/EPA in a 1:1 ratio over 6 months, supported by a healthy diet and moderate exercise, improved the liver function.

Fish oil also largely prevented the development of hepatic steatosis in a mouse model of hepatic steatosis (Kalish et al., 2013).

US2013/156861 A1 discloses that the oral administration of krill oil may mitigate alcoholic liver injury.

The oral supplementation of Epanova^{™} showed beneficial effects in a mouse model of nonalcoholic steatohepatitis (Cazanave et al. 2016).

WO2015/053379 discloses a method for treating fatty liver disease comprising the administration of EPA ethyl ester.

WO2016/057915 A1 describes a beneficial effect of the oral administration of DHA in a NASH mouse model.

In summary, there is data regarding the effects of EPA and DHA in liver disease. However, the focus of these studies was either a long-term treatment over 3-6 months which is not ideal concerning patient compliance, especially in an indication which would not have occurred if the patient had followed some measures like alcohol abstinence and a healthy diet, or the use of a commercially available emulsion resulting in unusable dose requirements. Also, so far, there has not been any particular focus on the specific and particularly beneficial ratios of EPA to DHA or the parenteral route of administration in this respect.

Therefore, there is still a need for the development of an improved therapy for patients suffering from liver disease.

The technical problem underlying the present invention can be seen as the provision of means and methods for complying with the aforementioned needs. The technical problem is solved by the embodiments characterized in the claims and herein below.

The present invention is defined in the appended claims. Any embodiments which do not fall under the scope of the claims are not the subject of the present invention.

Accordingly, the present invention relates to a composition for use in treating and/or preventing a liver disease in a subject, wherein said composition comprises eicosapentaenoic acid (EPA) and docosahexaenoic acid (DHA) in a weight ratio of between 2.0 (EPA) to 1.0 (DHA) and 10.0 (EPA) to 1.0 (DHA).

The term "composition" as used herein refers to any mixture of EPA and DHA in the aforementioned weight ratio. The composition shall be suitable for use in the treatment and/or prevention of said disease. Typically, such a composition is provided in a pharmaceutically acceptable form, e.g., being essentially free of harmful contaminants. Accordingly, all ingredients of the composition are provided in a purity grade sufficient for pharmaceutical applications. Preferably, the composition is formulated under GMP standardized conditions or the like in order to ensure quality, pharmaceutical safety, and effectiveness of the composition until application. Preferably, the composition according to the present invention may be, thus, formulated as a medicament. Said medicament is, typically, to be adapted to the mode of administration including its formulation as well as packaging. Dosage recommendations as those specified elsewhere herein in detail shall be, preferably, indicated in the prescribers or users instructions.

The term "liver disease" as used herein refers to any condition which impairs the physiological liver function. Typically, the disease is selected from alcoholic or nonalcoholic fatty liver disease, especially liver steatosis, steatohepatitis, liver inflammation, liver fibrosis, liver cirrhosis and liver cancer, preferably hepatocellular adenoma (HCA) or a hepatocellular carcinoma (HCC). Preferably, said liver disease is a non-alcoholic fatty liver disease (NAFLD), especially non-alcoholic steatohepatitis (NASH), liver steatosis, liver inflammation, liver fibrosis, liver cirrhosis and liver cancer, preferably hepatocellular adenoma (HCA) or a hepatocellular carcinoma (HCC). The symptoms and clinical signs of these liver diseases are all well known in the art and described in standard text books of medicine in great detail. The diagnosis of liver diseases may typically encompass the measurement of biochemical parameters such as liver enzymes, imaging of the liver by, e.g., ultrasound, CT or MRT techniques, and/or histological analysis of liver tissue obtained by liver biopsies.

The composition to be used according to the present invention shall comprise eicosapentaenoic acid (EPA). EPA is a long-chain, polyunsaturated fatty acid also known as (5Z,8Z,11Z,14Z,17Z) - 5,8,11,14,17 - icosapentaenoic acid or 20:5(n-3). EPA is an omega-3 fatty acid with a 20-carbon chain and five cis double bonds; the first double bond is located at the third carbon from the omega end. Its chemical structure is also well known in the art and described, e.g., under CAS number 10417-94-4.

Moreover, in addition to EPA, the composition to be used in accordance with the present invention shall further comprise docosahexaenoic acid (DHA). DHA is a long-chain, polyunsaturated fatty acid also known as (4Z,7Z,10Z,13Z,16Z,19Z)-docosa-4,7,10,13,16,19 - hexaenoic acid or 22:6(n-3). Docosahexaenoic acid is a 22-carbon chain with six cis double bonds, the first double bond being located at the third carbon from the omega end. Its chemical structure is also well known in the art and described, e.g., under CAS number 6217-54-5.

The composition according to the present invention shall comprise EPA in an excessive amount compared to DHA, i.e. in a ratio of at least 2:1. Preferably, the weight ratio of EPA to DHA shall be between a ratio of 2.0 (EPA) to 1.0 (DHA) and 10.0 (EPA) to 1.0 (DHA), especially between a ratio of 4.5 (EPA) to 1.0 (DHA) and 6.0 (EPA) to 1.0 (DHA), for example 4.5 to 1.0, 5.0 to 1.0, 5.5 to 1.0, or 6.0 to 1.0.

EPA and/or DHA are, preferably, esterified in the composition according to the present invention. Thus, EPA occurs as EPA- containing triglyceride and DHA occurs as DHA-containing triglyceride. However, in another embodiment, the invention also encompasses compositions comprising EPA and DHA as free fatty acids or derivatives other than EPA or DHA triglyceride esters.

It is known that DHA and EPA and the derivatives thereof are contained as free fatty acids or esterified in the form of glycerides and in the form of other derivatives, in natural fats and oils, particularly, in fats and oils of aquatic animals (e.g., fish, marine mammals, and crustaceans such as krill and other euphausiids), animal tissues (e.g., brain, liver, eyes, etc.) and animal products such as eggs or milk. Thus, for example, they may be extracted from animal sources including aquatic animals (e.g., fish, marine mammals, and crustaceans such as krill and other euphausiids), animal tissues (e.g., brain, liver, eyes, etc.) and/or animal products such as eggs or milk.

Some methods for the isolation of docosahexaenoic acid (DHA) and eicosapentaenoic acid (EPA) and their derivatives and their conversion to pure docosahexaenoic acid (DHA) triglycerides and eicosapentaenoic acid (EPA) triglycerides are described in the art. Isolation by purification can be achieved by any means known to those of skill in the art and can include the extraction, e.g. by supercritical fluid extraction, of an oil from an organism which produces DHA and/or EPA and the subsequent purification via chromatographic methods. Alternatively, the oils can be extracted using extraction techniques such as described in WO2015/113987.

The composition according to the present invention, preferably, is an oil in water emulsion and, therefore, comprises and aqueous phase and an oil phase. The oil phase, preferably, amounts to 5 to 30 % by weight of the composition or, more preferably, amounts to 5 to 25% by weight, 5 to 20% by weight, 5 to 15% by weight 5 to 10% by weight or 9 to 10% by weight. More preferably, the oil phase of said composition amounts to at least 10% by weight, at least 15% by weight, at least 20% by weight, at least 25% by weight or at least 30% by weight of the total composition.

The aqueous phase of the composition according to the invention, preferably, comprises water having a purity grade suitable for intravenous administration. The amount of water is, preferably, in the range of from 95 to 70% by weight , preferably, 95 to75% by weight , more preferably, 95 to 80% by weight , more preferably, 90 to 80% by weight . Preferably, the composition according to the invention is an emulsion, in particular an oil-in-water emulsion. In case the emulsion is an oil-in-water emulsion, the oil droplets, preferably, have a mean droplet size (D_{4,3}) in the range of from 0.1 µm to 0.3 µm, preferably, of from 0.15 µm to 0.25 µm when measured with an LS 13 320 Laser Diffraction Particle Size Analyser (Beckman Coulter) according 10 to USP <729>.

EPA and DHA are, preferably, present in an amount of at least 65% based on the total weight of the oil phase. In other words, EPA and DHA are present in the composition according to the invention in an amount of at least 65% by weight of the oil phase, such as from 65% to 95% by weight of the oil phase, more preferably, at least 70% by weight of the oil phase, more preferably, at least 75% by weight of the oil phase, more preferably, at least 80% by weight of the oil phase, more preferably, of from 85 to 90% by weight of the oil phase.

Moreover, also preferably, the total amount of EPA and DHA in the composition according to the present invention is between 10 to 100 mg/ml, especially 40 to 90 mg/ml, more preferably between 60 to 80 mg/ml.

Preferably, the oil phase present in the composition according to the present invention comprises less than 1% by weight, more preferably less than 0.5% by weight, more preferably less than 0.1% by weight, more preferably less than 0.05% by weight, more preferably less than 0.01% by weight, more preferably essentially no, more preferably no, medium chain fatty acid derivatives. This amount refers to the sum of all medium chain fatty acid derivatives present and is based on the total weight of the oil phase. The term "essentially no" in this context refers to an amount < 0.01% by weight including 0% by weight. The medium chain fatty acid derivative as referred to herein are fatty acid derivatives, such as mono-, di- or triglycerides (MCT), comprising a medium chain fatty acid or alkyl esters of medium chain fatty acids these fatty acids being 6 to 12 carbon atoms in length. Medium chain fatty acids include but are not limited to caproic acid, caprylic acid, capric acid and lauric acid. Due to the fact that MCTs may be omitted, it has been found that stable compositions may be provided without these medium chain fatty acid derivatives which may comprise an even higher amount of EPA derivatives and DHA derivatives.

The composition according to the present invention may further comprise additional components. Preferably, it comprises at least one amphoteric surfactant, at least one co-surfactant and at least one co-solvent.

A surfactant as referred to herein relates to compounds which stabilize the composition by reducing the interfacial tension between the oil phase and the water phase and which typically comprise at least one hydrophobic group (their tail) and at least one hydrophilic group (their head). These surfactants (which may also be referred to as emulsifiers) are, preferably, used in amounts effective to provide, optionally, together with further surfactants present, stable and even distribution of the oil phase within the aqueous phase. In particular, these surfactants are selected from surfactants which have been approved for parenteral administration.

The term "amphoteric surfactant" refers to surfactants which carry a charge that varies depending on the pH of the solution. At low pH (acidic conditions), they act as cationic surfactants while at high pH (basic), they act as anionic surfactants. When both charge groups are permanent, the surfactants are sometimes also called zwitterionic.

More preferably, the at least one amphoteric surfactant is lecithin. Within the meaning of the present invention the term "lecithin" refers to a naturally occurring or synthetic lecithin that may be suitably refined. Suitable lecithins include, but are not limited to, lecithins derived from egg, corn or soybean or mixtures thereof. Further suitable lecithins include, but are not limited to, dihexanoyl-L-alphalecithin, dioctanoyl-L-alpha-lecithin, didecanoyl-L-alpha-lecithin, didodecanoyl-L-alpha-lecithin, ditetradecanoyl-L-alpha-lecithin, dihexadecanoyl-L-alpha-lecithin, dioctadecanoyl-L-alpha-lecithin, dioleoyl-L-alpha-lecithin, dilinoleoyl-L-alpha-lecithin and alpha-palmitol. Lecithins are typically mixtures of diglycerides of fatty acids linked to the choline ester of phosphoric acid and can contain differing amounts of other compounds depending on the method of isolation. Typically, commercial lecithin is a mixture of acetone-insoluble phosphatides.

Preferably, the lecithin is obtained from egg or from seeds including soybean and corn, using methods well known in the art. Lecithin obtained from soybean is referred to herein as soy lecithin. Lecithin obtained from egg is referred to herein as egg lecithin. Preferably, the composition comprises lecithin as amphoteric surfactant, more preferably the lecithin is selected from the group consisting of egg lecithin, soy lecithin, and mixtures thereof. As to the soy lecithin, said soy lecithin typically comprises at least 50% by weight of phospholipids, more preferably, of from 50 to 95% by weight, more preferably, of from 70 to 80% by weight and, most preferably, of from 75 to 85% by weight, based on the total weight of the soy lecithin.

The soy lecithin, as described above, usually comprises at least phosphatidylcholine and phosphatidylethanolethanolamine, and usually further comprises phosphatidylinositol and phosphatidic acid. A typical composition comprises phosphatidylcholine in an amount in the range of from 70% by weight 10 to 80% by weight and phosphatidylethanolethanolamine in an amount in the range of from 5 to 10% by weight, based on the total weight of the soy lecithin. Such soy lecithin is commercially available, for example as Epikurin^{™}170.

As to the egg lecithin, said egg lecithin typically comprises at least 50% by weight of phospholipids, preferably, at least 80% by weight, more preferably, at least 90% by weight, based on the total weight of the egg lecithin. The egg lecithin, as described above, usually also comprises phosphatidylcholine, phosphatidylethanolethanolamine, phosphatidylinositol and phosphatidic acid. A typical composition comprises phosphatidylcholine in an amount in the range of from 60 to 85% by weight and phosphatidylethanolethanolamine in an amount in the range of from 7 to 18% by weight, based on the total weight of the egg lecithin. Such egg lecithins are commercially available, for example as PL 90 or Lipoid E80. It is to be understood that lecithin may be employed in combination with other amphoteric surfactants. Preferably, the composition only comprises lecithin as amphoteric surfactant.

The total amount of amphoteric surfactants within the composition is, preferably, in the range of from 0.5 to 5% by weight, more preferably 0.75 to 3% by weight, more preferably, in the range of from 1% by weight to 2% by weight, such as 1.0, 1.1, 1.2, 1.3, 1.4, 1.5, 1.6, 1.7, 1.8, 1.9 or 2.0 % by weight, based on the total weight of the composition.

The composition, preferably, comprises at least one co-surfactant. A co-surfactant is an amphiphilic molecule, i.e. a molecule that contains both hydrophilic and lipophilic groups. Usually a co-surfactant substantially accumulates with the surfactant at the interfacial layer. The hydrophile-lipophile balance (HLB) number is used as a measure of the ratio of hydrophilic and lipophilic groups present in a surfactant or co-surfactant, respectively. Usually, a co-surfactant with a very low HLB value (thus with a relatively high affinity to oil) is used together with a surfactant with a high HLB to modify the overall HLB of the system. Unlike the surfactant, the co-surfactant is usually not capable of forming selfassociated structures, like micelles, on its own. Several kinds of molecules including nonionic surfactants, alcohols, amines and acids, can function as co-surfactants in a given system. The quantity of a co-surfactant in a system is usually less than that of the surfactant and it often serves to modify the overall HLB value of the system. The co-surfactant has the effect of further reducing the interfacial tension, whilst increasing the fluidity of the interface. Co-surfactants may also adjust the curvature of the interfacial film by partitioning between the tails of the surfactant chains, allowing greater penetration of the oil between the surfactant tails.

Preferably, the at least one co-surfactant is an unsaturated fatty acid, preferably an omega-9 fatty acid, more preferably a monounsaturated omega-9 fatty acid and, more preferably, oleic acid. It has been found that emulsions comprising oleic acid in combination with a co-solvent and an amphoteric surfactant are particularly stable.

The total amount of at least one co-surfactant is, preferably, in the range of from 0.01 to 1% by weight, more preferably, in the range of from 0.02% by weight to 0.5 % by weight, more preferably, in the range of from 0.03% by weight to 0.25% by weight, based on the total weight of the composition. Preferably, the composition comprises less than 0.03% by weight of sodium oleate based on the total weight of the composition. More preferably, the composition comprises less than 0.02% by weight, even more preferably less than 0.01% by weight sodium oleate.

It is noted that the composition described above may comprise any other suitable surfactant or any other co-surfactant provided that the composition comprises less than 0.03% by weight of sodium oleate based on the total weight of the composition. As suitable other surfactants may be, e.g., nonionic or anionic surfactants.

Preferably, the composition comprises less than 0.02% by weight, more preferably less than 0.01% by weight, more preferably essentially no sodium oleate, preferably no sodium oleate.

The term "essentially no" means that essentially no, i.e. an amount of < 0.01% by weight including 0% by weight, sodium oleate, preferably, 0% by weight, is added to the composition during the preparation process. It is noted that in case oleic acid is added to the mixture during the preparation of the composition, and in case any further sodium salts, such as sodium hydroxide, are added during the preparation process, it may not be ruled out that at least a portion of this oleic acid is being transformed into its corresponding sodium salt, i.e. into sodium oleate. However, it is envisaged that substantially all of the oleic acid should be present in the oil phase and that should thus not be transformed to the corresponding sodium salt. Such minor amount of sodium oleate which may be formed is, thus, included in term "essentially no".

The term co-solvent refers to molecules that may increase the stability of the composition according to the invention. In addition to making the environment more hydrophobic by reducing the dielectric constant of water, co-solvents increase the amount of molecularly dispersed surfactant in the aqueous phase. Availability of free surfactant aids in the solubilisation of hydrophobic molecules can be achieved by creating pockets of hydrophobic regions within the aqueous phase. Examples of co-solvents include ethanol, glycerin, propylene glycol and polyethylene glycol (PEG).

Preferably, the at least one co-solvent is a polyalkylene glycol or an alkylene glycol, preferably, polyethylene glycol or propylene glycol, more preferably, polyethylene glycol.

Preferably, the co-solvent is selected from the group consisting of: PEG 200, PEG 300, PEG 400, 10 PEG 600, PEG 1000, PEG 1450, PEG 3350, PEG 4000, PEG 6000, PEG 8000 and PEG 20000. Most preferably, the co-solvent is PEG 400.

Preferably, the total amount of co-solvents present ranges from 0.1 to 2.0% by weight, more preferably, from 0.25 to 1.75% by weight, more preferably, from 0.50 to 1.50% by weight, more preferably, from 0.70 to 1.40% by weight, more preferably, from 0.80 to 1.30% by weight, and even more preferably, from 0.90 to 1.20% by weight, based on the total weight of the composition. It has been found that emulsions comprising polyethylene glycol and/or propylene glycol as well as lecithin are particularly stable.

The composition as described above, preferably, comprises at least one amphoteric surfactant, at least one co-surfactant and at least one co-solvent, wherein at least one co-surfactant is oleic acid, and wherein the composition comprises less than 0.03 %, more preferably less than 0.01 % by weight of sodium oleate based on the total weight of the composition.

Also preferably, the composition according to the present invention comprises at least one amphoteric surfactant, at least one co-surfactant and at least one co-solvent, wherein the composition comprises less than 0.03 % by weight of sodium oleate, based on the total weight of the composition, and wherein the at least one co-solvent is a polyalkylene glycol or an alkylene glycol, preferably polyethylene glycol or propylene glycol, more preferably polyethylene glycol.

Yet preferably encompassed according to the present invention is a composition comprising at least one amphoteric surfactant, at least one co-surfactant and at least one co-solvent, wherein the at least amphoteric surfactant is lecithin, and wherein the co-solvent is polyethylene glycol and/or propylene glycol, and wherein the composition comprises less than 0.03 % by weight of sodium oleate based on the total weight of the composition.

Preferably, the composition as described above comprises oleic acid as co-surfactant, lecithin as amphoteric surfactant and polyethyleneglycol and/or propylene glycol as co-solvent(s). It is to be understood that polyethylene glycol may be employed in combination with other co-solvents such as any one of the co-solvents mentioned above. Preferably, the composition only comprises polyethylene glycol and/or propylene glycol as co-solvent. In case polyethylene glycol is employed as co-solvent, the polyethylene glycol, preferably, has a mean molecular weight in the range of from 100 to 20000 Da, more preferably, in the range of from 200 to 1000 Da, more preferably, in the range of from 300 to 600 Da, most preferably, around 400 Da.

The composition to be used in accordance with the present invention may comprise further ingredients and, in particular, at least one tonicity agent, at least one antioxidant and/or at least one additive.

Preferably, the composition according to the invention comprises at least one tonicity agent. Tonicity agents are substances which are used to confer tonicity to, e.g., pharmaceutical compositions. A tonicity agent useful in the present composition can be any pharmaceutically acceptable tonicity agent. Common tonicity agents include, but are not limited to, agents selected from the group consisting of sodium chloride, mannitol, lactose, dextrose (hydrous or anhydrous), sucrose, glycerol, sorbitol, and solutions of the foregoing. Preferably, the tonicity agent is glycerol.

If present, preferably the total amount of tonicity agents present is in the range of 0 to 10% by weight, more preferably, from 1 to 5% by weight, more preferably, from 1 to 4% by weight, more preferably, from 1 to 3% by weight, more preferably, from 1.5 to 2.8% by weight, and, even more preferably, from 2.0 .to 2.5% by weight, based on the total weight of the composition.

Preferably, the composition has an osmolality in the range 305 to 420 mOsmol/kg, more preferably in the range of from 300 to 420 mOsmol/kg, measured with a Vapor Pressure Osmometer, Model 5520 (Vapro TM) according to USP <785>.

Preferably, the composition according to the invention comprises at least one antioxidant, i.e., an agent with antioxidant activity and, preferably, at least two agents with antioxidant activity. An antioxidant useful in the present composition can be any pharmaceutically acceptable compound having antioxidant activity including sodium metabisulfite, sodium bisulfite, sodium sulfite, sodium thiosulfate, sodium formaldehyde sulfoxylate, sodium formaldehyde bisulfite, thioglycerol, thiosorbitol, thioglycolic acid, cysteine hydrochloride, n-acetyl-cysteine, citric acid, alpha-tocopherol, betatocopherol, gamma-tocopherol, Trolox (soluble form of vitamin E), butylated hydroxyanisole (BHA), butylated hydroxytoluene (BHT), t-butylhydroquinone (TBHQ), monothioglycerol, propyl gallate, lopurinol, carnosine, histidine, enzymes, such as superoxide dismutase, catalase, selenium glutathione peroxidase, phospholipid hydroperoxide and glutathione peroxidase, Coenzyme Q 10, tocotrienols, carotenoids, quinones, bioflavonoids, polyphenols, bilirubin, ascorbic acid, isoascorbic acid, uric acid, metal-binding proteins, ascorbic acid palmitate, an antioxidant obtained or obtainable from rosemary and rosemary extract.

The at least one agent with antioxidant activity, preferably, is tocopherol, even more preferably, it is selected from the group consisting of: alpha-tocopherol, beta-tocopherol, gamma-tocopherol, ascorbic acid, and mixtures of two or more thereof.

If present, the total amount of agents with antioxidant activity is preferably in the range of from 0.01 to 0.05% by weight, more preferably from 0.01 to 0.04% by weight, more preferably from 0.01 to 0.03% by weight, and even more preferably from 0.015 to 0.025% by weight, based on the total weight of the composition.

More preferably, the composition comprises at least two different agents with antioxidant activity. For example, the present invention comprises alpha-tocopherol and betatocopherol, or alpha tocopherol and gamma-tocopherol, or beta-tocopherol and gamma-tocopherol, or alpha-tocopherol and ascorbic acid, or beta- tocopherol and ascorbic acid, or gamma-tocopherol and ascorbic acid. According to a further preferred embodiment, the present invention comprises a mixture of beta tocopherol, alpha-tocopherol and gamma-tocopherol.

It is to be understood that other physiologically safe additives may also be present in the composition according to the invention including, but not limited to, salts commonly used for intravenous application such as sodium chloride and nonelectrolytes such as glucose, pH modifiers (such as acetic acid and sodium acetate) and buffers (such as acetate, lactate, and phosphate buffer systems composed of the acid and a salt of the acid) as well as selenium compounds.

One skilled in the art will understand that the pH of the composition may, for example, be adjusted through the use of buffers, such as phosphate buffers, or neutralization agents, such as sodium hydroxide. Preferably, the composition according to the present invention has a pH value close to physiological pH or above since it is contemplated that at such pH values the fatty acids are less prone to peroxidation. The final pH of the composition is, preferably, in the range of from 7.0 to 10, more preferably, in the range of from 8 to 10.

By way of example, the composition may further comprise other additives conventionally used in pharmaceutical compositions. Such additives include carbohydrate nutrients, electrolytes, amino acids, vitamins, trace minerals, preservatives, anti-foaming agents, buffering agents, chelating agents, and mixtures thereof. The amounts of such additives can be readily determined by one skilled in the art according to the particular properties desired. The components of the composition according to the present invention described above can be combined freely as long as no apparent inconsistencies result. Especially preferred embodiments of the composition according to the invention are still explicitly described in the following.

Further details of the composition for use in accordance with the present invention are also found in WO2015/113987.

Surprisingly, it has been found that parenteral administration of the composition results, next to a significantly faster onset of action, within a timeframe of less than one month to a long-lasting effect.

The composition according to the present invention may be administered by any suitable mode of administration allowing the composition and its active ingredients to become therapeutically effective. Depending on the formulation of the composition, the skilled person is well aware of such suitable administration modes. Preferably, however, the composition is to be administered parenterally or enterally. More preferably, the composition is to be administered parenterally, e.g. intraveneously, subcutaneously, intraperitoneally, intramuscularly. Particularly preferred is intravenous administration.

The composition according to the present invention is suitable for long-term administration as well as for administration within limited time windows. The composition may be administered once as a bolus or continuously. When administered continuously, the composition may be applied at different times in separate dosages or may be permanently applied, e.g., via infusions.

In particular, the composition may be, preferably, administered shortly after the onset or diagnosis of the disease. However, once administered within this time window, the composition may elicit a long-term therapeutic effect in the subject. Preferably, said composition is, thus, to be administered within a time window of four weeks after onset or diagnosis of said liver disease, preferably within three weeks, two weeks or one week.

In one embodiment of the invention, the composition is not administered to the subject again after said time window, at least as long as the long-lasting therapeutic effect is effective.

Within the aforementioned time window of one to four weeks, the composition may be administered once or several times (e.g., at least two, three, four, five, six, seven, eight, nine or ten times in total) per week or in total. Surprisingly, it has been found that administration of the emulsion according to the invention up to three times per week already reaches a beneficial effect on liver diseases, for example, three times per week within three weeks after onset or diagnosis of said liver disease.

The composition may be administered in an amount of 10 to 5,000 µl/kg body weight per day and, more preferably, in an amount of 10 to 1,000 µl/kg body weight per day. More preferably, the total amount of EPA and DHA present in the composition according to the present invention is between 10 to 100 mg/ml, especially 40 to 90 mg/ml, especially between 60 to 80 mg/ml. The total amount of EPA and DHA administered per kg body weight per day is, preferably, in the range of 400 µg to 400 mg, 1 mg to 200 mg or in the range of 1 mg to 100 mg or 1 mg to 60 mg. It has been found that the beneficial effect of the composition according to the invention is reduced for daily dosages of > 100 mg/kg body weight. Therefore, the total amount of EPA and DHA administered per kg body weight by day is preferably below 100 mg.

Advantageously, it has been found in the studies underlying the present invention that a composition and, in particular, an oil in water emulsion, comprising EPA and DHA in a certain weight ratio, elicits a therapeutic effect on liver diseases. This therapeutic effect was experimentally shown to be particularly pronounced and long-lasting when the composition was administered at an early stage of the experiment. In particular, the repeated dose treatment using a composition comprising EPA to DHA wherein the composition contains EPA and DHA in a ratio of between 2.0:1 to 10.0:1.0, especially 4.5 to 1.0 to 6.0 to 1.0 during the first three weeks after the onset of the disease in preclinical liver disease models for NASH resulted in a significant and long lasting improvement of the disease. For example, liver injury parameters such as ALT and AST in the plasma were reduced as well as hepatic cholesterol, triglycerides and liver weight. The ballooning of hepatocytes, the liver inflammation and the liver fibrosis as well as the NAS score describing the extent of liver steatosis were also reduced. In yet another preclinical disease model, histological analysis of the liver revealed that the incidence of liver cancer and the incidence and severity of fat infiltration and foci of altered hepatocytes are reduced after the treatment.

A comparison with EPA and DHA containing compositions including the commercially available Omegaven^{®} 10% (Fresenius Kabi, Deutschland GmbH) demonstrated that the aforementioned beneficial therapeutic effects, surprisingly, can only be observed in the dose range below 400 mg/kg body weight per day EPA and DHA wherein the ratio of EPA to DHA is between 2.0 to 1.0 and 10.0 to 1.0, especially between 6.0 to 1.0 and 4.5 to 1.0.

The treatment regime can be, surprisingly, also limited to a short time window after the onset or diagnosis of the disease due to its long-lasting effects. Long-lasting effects, in the context of this invention, can be several months up to 6 months. In another embodiment, the effect lasts up to 12 months. Nevertheless, a long-term treatment with the composition is also feasible. Thus, thanks to the present invention a therapy can be provided for liver disease which safeguards high quality in the life of the patients.

The explanations and comments made herein above apply mutatis mutandis for the preferred embodiments of the invention described in the following.

### Method of manufacturing

The composition for use according to the present invention may be, preferably, manufactured by a method comprising the steps of:
(a) providing an aqueous phase comprising the at least one co-solvent and the at least one amphoteric surfactant,
(b) providing an oil phase comprising omega-3 fatty acid triglycerides selected from the group consisting of eicosapentaenoic acid triglyceride, docosahexaenoic acid triglyceride and mixtures thereof, and
(c) mixing the oil phase according to (b) with the aqueous phase according to (a), wherein the at least one co-surfactant is added either in step (b) or in step (c), and wherein less than 0.03 % by weight of sodium oleate based on the total weight of the final composition are added during the method.

It is to be understood that any one of the optional further components of the composition may be added in any one of steps (a) to (c), or in one or more additional steps.

Step (a) is, preferably, carried out by mixing the at least one co-solvent and the at least one amphoteric surfactant together or subsequently with water. This step, preferably, is carried out at a temperature in the range of from 25 to 70 °C, wherein during this step, the temperature may be varied or held essentially constant. Preferably, initially, the at least one co-solvent is mixed with water. Preferably, subsequently, the at least one amphoteric surfactant is added to the mixture comprising water and the at least one co-solvent thereby forming a dispersion. Preferably, the resulting mixture is mixed for example with a high shear mixer. Preferably, the mixture is then heated to a temperature in the range of from 40 to 70°C, preferably, 50 to 65°C, more preferably, 55 to 60°C, preferably for a time in the range of from 1 min to 2 h, more preferably, of from 5 min to 1 h, more preferably, of from 10 min to 15 min.

It is to be understood that in step (a) further additives may be added. For example, in case the composition comprises at least one tonicity agent, this may be, in principle, added in any step of the method described above. According to one preferred embodiment, this additive, if present, is added in step (a). Thus, preferably, step (a) further comprises mixing at least one tonicity agent with water, more preferably mixing glycerol with water. These additives may mixed with water prior to or after the addition of the at least one co-solvent and/or the at least one amphoteric surfactant. More preferably, these additives are mixed with water prior to or after the addition of the at least one co-solvent.

Preferably, step (a) further comprises adjusting the pH of the aqueous phase, such as through the use of buffers, such as phosphate buffers, or neutralization agents, such as sodium hydroxide, to a desired pH which is, preferably, in the range of from 7.0 to 10, more preferably in the range of from 8 to 10.

In step (b), as outlined above, initially a mixture comprising EPA and DHA triglycerides is provided, wherein the EPA and DHA triglycerides may be obtained by any way known to those skilled in the art. Preferably, the oil phase is heated in step (b), that is prior to step (c), to a temperature in the range of from 30 to 70°C, more preferably, from 40 to 65°C, more preferably, from 50 to 60°C, more preferably, to a temperature around 55°C, preferably, for a time in the range of from 1 min to 30 min, more preferably, from 3 min to 20 min, more preferably, from 5 min to 15 min. Preferably, in step (b), the at least one co-surfactant is added. In case the oil phase is heated, the co-surfactant may be added prior to, during or after the heating step. Preferably, the co-surfactant is added during the heating step. The oil phase is, preferably, homogenized, preferably at a temperature in range of from 30 to 70°C, more preferably, from 40 to 65°C, more preferably, from 50 to 60°C, more preferably, to a temperature around 55°C.

According to a preferred embodiment, at least one agent with antioxidant activity, if present, is added in step (b). Thus, in this case, in step (b), optionally, at least one co-surfactant and, optionally, at least one agent with antioxidant activity are added to the mixture of EPA triglycerides and DHA triglycerides, more preferably, oleic acid and/or at least one tocopherol are added in step (b). Thus, step (b), preferably, comprises providing an oil phase by mixing eicosapentaenoic acid triglycerides and docosahexaenoic acid triglycerides with the at least one co-surfactant and/or the at least one agent with antioxidant activity, wherein at least 60% by weight of the oil phase consist of eicosapentaenoic acid triglycerides and docosahexaenoic acid triglycerides. Alternatively, the provided mixture comprising EPA and DHA triglycerides may already comprise the or at least part of the total amount of the at least one co-surfactant and/or the at least one agent with antioxidant activity.

In step (c), the method further comprises mixing the oil phase according to (b) with the aqueous phase according to (a) to give a mixture of oil phase and an aqueous phase. Preferably, thereby a pre- emulsion or an emulsion is formed. The mixing may be carried out by any methods known to those skilled in the art. Preferably, the mixing is carried out using a high shear mixer.

Preferably, the oil phase is added to the aqueous phase or vice versa at a temperature in the range of from 50 to 70°C, more preferably from 55 to 65°C. Preferably, the oil phase is added to the aqueous phase or vice versa at a pressure, such as under nitrogen pressure, in the range of from 0.20 to 0.80 bar, more preferably, from 0.20 to 0.40 bar, such as at around 0.30 bar. During this step, pressure may be varied or held essentially constant. According to a preferred embodiment, the mixture is stirred for a time in the range of from 1 min to 1 h, preferably of from 10 min to 30 min, to give a pre-emulsion. During this step, the temperature may be varied or held essentially constant. It is to be understood that further components may also be added after the formation of the pre-emulsion. According to a preferred embodiment, the pH of the pre-emulsion is adjusted to a pH in the range of from 8 to 10, in particular by adding sodium hydroxide, if necessary.

In step (d), preferably, the method further comprises the homogenization of the mixture obtained from step (c). This homogenization may be carried out by any suitable methods known to those skilled in the art. Preferably, the mixture is homogenized at temperature in range of from 40 to 70°C, more preferably, from 50 to 70°C, more preferably, from 50 to 60°C. Preferably, the mixture is homogenized at a pressure in the range of from 400 to 600 bar, more preferably, from 450 to 550 bar. During this step, the pressure may be varied or held essentially constant. During this step, the pressure may be varied or held essentially constant. Preferably, the homogenization may for example be carried out using a high pressure homogenizer or a microfluidizer.

Thus, the present invention also relates to a method as described above for preparing the inventive composition, the method further comprising:
(d) homogenizing the mixture, preferably the pre-emulsion, obtained from (c) at a temperature in the range of from 50 to 60°C and at a pressure at a pressure in the range of from 400 to 600 bar.

After the homogenization step, further steps may be carried out, such as purification steps or filtration steps.

In a further step (e), preferably, the composition obtained in (c) or (d) is heat treated, more preferably sterilized, to ensure its suitability for parenteral administration. The sterilization may be carried out by any suitable methods known to those skilled in the art. In particular, the sterilization is carried out by autoclaving, preferably, at a temperature in the range of from 119°C to 122°C, more preferably, at a temperature of around 121°C, preferably, for a time in the range of from 1 min to 30 min, preferably, of from 10 min to 15 min.

Thus, the present invention also relates to a method as described above for preparing the composition of the invention further comprising:
(e) autoclaving the mixture obtained from (c) or (d), preferably, from (d), at a temperature in the range of from 119°C to 122°C for a time in the range of from 10 min to 15 min.

Further details of how to manufacture the composition for use in accordance with the present invention are also found in WO2015/113987.

It is to be understood that the preparation of the composition of the invention, preferably, takes place under GMP standardized conditions in order to ensure quality, safety and effectiveness of the composition when used as a medicament or in parenteral nutrition. Further criteria for an ingredient or a composition being pharmaceutically acceptable can be derived from approval regulations by a regulatory agency and/or generally recognized pharmacopoeias.

### FIGURES

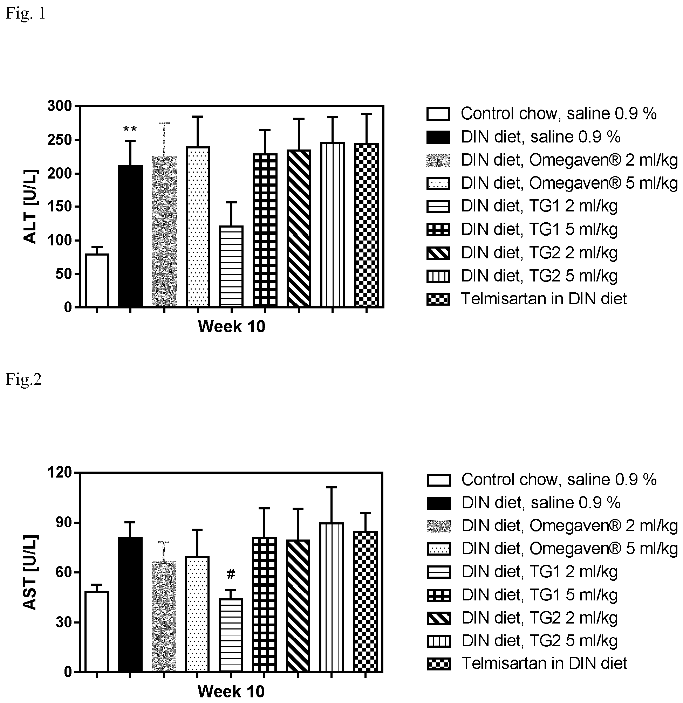

**Figure** 1 shows alanine transaminase (ALT, mean of all animals + standard error of the mean) plasma levels of mice with diet induced (DIN) non-alcoholic steatohepatitis (NASH) and controls 10 weeks after initiation of treatment as observed in Example 1. The respective treatments are indicated by the following symbols. White bar: healthy control animals which received 0.9 % saline, indicated as "Control chow, saline 0.9%". Black bar: animals with diet induced NASH which received 0.9 % saline, indicated as "DIN diet, saline 0.9 %". Gray bar: animals with diet induced NASH which received 2 ml/kg Omegaven^{®}, indicated as "DIN diet, Omegaven^{®} 2 ml/kg". Dotted bar: animals with diet induced NASH which received 5 ml/kg Omegaven^{®}, indicated as "DIN diet, Omegaven+ 5 ml/kg. Horizontal lines: animals with diet induced NASH which received 2 ml/kg TG1, indicated as "DIN diet, TG1 2 ml/kg". Grid: animals with diet induced NASH which received 5 ml/kg TG1, indicated as "DIN diet, TG1 5 ml/kg. Diagonal lines: animals with diet induced NASH which received 2 ml/kg TG2, indicated as "DIN diet, TG2 2 ml/kg". Vertical lines: animals with diet induced NASH which received 5 ml/kg TG2, indicated as "DIN diet, TG1 5 ml/kg. Checkered bar: animals with diet induced NASH which received telmisartan mixed in the feed, indicated as "Telmisartan in DIN diet". ** indicates a statistically significant difference to the control chow + 0.9% saline group (p<0.01, t-test).
**Figure 2** shows aspartate transaminase (AST, mean of all animals + standard error of the mean) plasma levels of mice with diet induced (DIN) non-alcoholic steatohepatitis (NASH) and controls 10 weeks after initiation of treatment as observed in Example 1. The respective treatments are indicated by the following symbols. White bar: healthy control animals which received 0.9 % saline, indicated as "Control chow, saline 0.9%". Black bar: animals with diet induced NASH which received 0.9 % saline, indicated as "DIN diet, saline 0.9 %". Gray bar: animals with diet induced NASH which received 2 ml/kg Omegaven^{®}, indicated as "DIN diet, Omegaven^{®} 2 ml/kg". Dotted bar: animals with diet induced NASH which received 5 ml/kg Omegaven^{®}, indicated as "DIN diet, Omegaven^{®} 5 ml/kg. Horizontal lines: animals with diet induced NASH which received 2 ml/kg TG1, indicated as "DIN diet, TG1 2 ml/kg". Grid: animals with diet induced NASH which received 5 ml/kg TG1, indicated as "DIN diet, TG1 5 ml/kg. Diagonal lines: animals with diet induced NASH which received 2 ml/kg TG2, indicated as "DIN diet, TG2 2 ml/kg". Vertical lines: animals with diet induced NASH which received 5 ml/kg TG2, indicated as "DIN diet, TG1 5 ml/kg. Checkered bar: animals with diet induced NASH which received telmisartan mixed in the feed, indicated as "Telmisartan in DIN diet". # indicates a statistically significant difference to the DIN diet + 0.9% saline group (p<0.05, t-test).

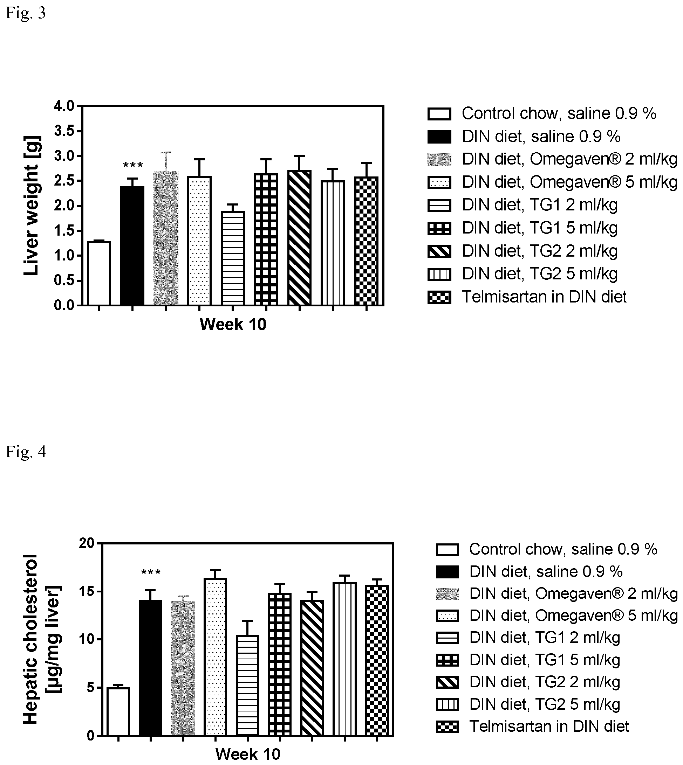

**Figure 3** shows absolute liver weight (mean of animals + standard error of the mean) of mice with diet induced (DIN) non-alcoholic steatohepatitis (NASH) and controls 10 weeks after initiation of treatment as observed in Example 1. The respective treatments are indicated by the following symbols. White bar: healthy control animals which received 0.9 % saline, indicated as "Control chow, saline 0.9%". Black bar: animals with diet induced NASH which received 0.9 % saline, indicated as "DIN diet, saline 0.9 %". Gray bar: animals with diet induced NASH which received 2 ml/kg Omegaven^{®}, indicated as "DIN diet, Omegaven^{®} 2 ml/kg". Dotted bar: animals with diet induced NASH which received 5 ml/kg Omegaven^{®}, indicated as "DIN diet, Omegaven^{®} 5 ml/kg. Horizontal lines: animals with diet induced NASH which received 2 ml/kg TG1, indicated as "DIN diet, TG1 2 ml/kg". Grid: animals with diet induced NASH which received 5 ml/kg TG1, indicated as "DIN diet, TG1 5 ml/kg. Diagonal lines: animals with diet induced NASH which received 2 ml/kg TG2, indicated as "DIN diet, TG2 2 ml/kg". Vertical lines: animals with diet induced NASH which received 5 ml/kg TG2, indicated as "DIN diet, TG1 5 ml/kg. Checkered bar: animals with diet induced NASH which received telmisartan mixed in the feed, indicated as "Telmisartan in DIN diet". *** indicates a statistically significant difference to the control chow + 0.9% saline group group (p<0.001, t-test).
**Figure 4** shows hepatic cholesterol concentration (mean of all animals + standard error of the mean) of mice with diet induced (DIN) non-alcoholic steatohepatitis (NASH) and controls 10 weeks after initiation of treatment as observed in Example 1. The respective treatments are indicated by the following symbols. White bar: healthy control animals which received 0.9 % saline, indicated as "Control chow, saline 0.9%". Black bar: animals with diet induced NASH which received 0.9 % saline, indicated as "DIN diet, saline 0.9 %". Gray bar: animals with diet induced NASH which received 2 ml/kg Omegaven^{®}, indicated as "DIN diet, Omegaven^{®} 2 ml/kg". Dotted bar: animals with diet induced NASH which received 5 ml/kg Omegaven^{®}, indicated as "DIN diet, Omegaven^{®} 5 ml/kg. Horizontal lines: animals with diet induced NASH which received 2 ml/kg TG1, indicated as "DIN diet, TG1 2 ml/kg". Grid: animals with diet induced NASH which received 5 ml/kg TG1, indicated as "DIN diet, TG1 5 ml/kg. Diagonal lines: animals with diet induced NASH which received 2 ml/kg TG2, indicated as "DIN diet, TG2 2 ml/kg". Vertical lines: animals with diet induced NASH which received 5 ml/kg TG2, indicated as "DIN diet, TG1 5 ml/kg. Checkered bar: animals with diet induced NASH which received telmisartan mixed in the feed, indicated as "Telmisartan in DIN diet". *** indicates a statistically significant difference to the control chow + 0.9% saline group (p<0.001, t-test).

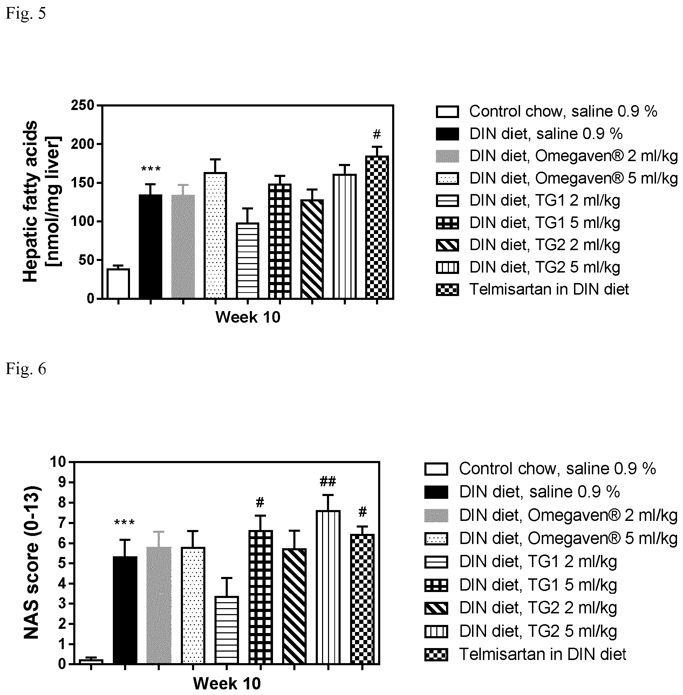

**Figure 5** shows hepatic fatty acid concentration (mean of all animals + standard error of the mean) of mice with diet induced (DIN) non-alcoholic steatohepatitis (NASH) and controls 10 weeks after initiation of treatment as observed in Example 1. The respective treatments are indicated by the following symbols. White bar: healthy control animals which received 0.9 % saline, indicated as "Control chow, saline 0.9%". Black bar: animals with diet induced NASH which received 0.9 % saline, indicated as "DIN diet, saline 0.9 %". Gray bar: animals with diet induced NASH which received 2 ml/kg Omegaven^{®}, indicated as "DIN diet, Omegaven^{®} 2 ml/kg". Dotted bar: animals with diet induced NASH which received 5 ml/kg Omegaven^{®}, indicated as "DIN diet, Omegaven^{®} 5 ml/kg. Horizontal lines: animals with diet induced NASH which received 2 ml/kg TG1, indicated as "DIN diet, TG1 2 ml/kg". Grid: animals with diet induced NASH which received 5 ml/kg TG1, indicated as "DIN diet, TG1 5 ml/kg. Diagonal lines: animals with diet induced NASH which received 2 ml/kg TG2, indicated as "DIN diet, TG2 2 ml/kg". Vertical lines: animals with diet induced NASH which received 5 ml/kg TG2, indicated as "DIN diet, TG1 5 ml/kg. Checkered bar: animals with diet induced NASH which received telmisartan mixed in the feed, indicated as "Telmisartan in DIN diet". *** indicates a statistically significant difference to the control chow + 0.9% saline group (p<0.001, t-test). # indicates a statistically significant difference to the DIN diet + 0.9% saline group (p<0.05, t-test).
**Figure 6** shows the non-alcoholic fatty liver disease scoring system (NAS) score (mean of all animals + standard error of the mean) of mice with diet induced (DIN) non-alcoholic steatohepatitis (NASH) and controls 10 weeks after initiation of treatment as observed in Example 1. The respective treatments are indicated by the following symbols. White bar: healthy control animals which received 0.9 % saline, indicated as "Control chow, saline 0.9%". Black bar: animals with diet induced NASH which received 0.9 % saline, indicated as "DIN diet, saline 0.9 %". Gray bar: animals with diet induced NASH which received 2 ml/kg Omegaven^{®}, indicated as "DIN diet, Omegaven^{®} 2 ml/kg". Dotted bar: animals with diet induced NASH which received 5 ml/kg Omegaven^{®}, indicated as "DIN diet, Omegaven^{®} 5 ml/kg. Horizontal lines: animals with diet induced NASH which received 2 ml/kg TG1, indicated as "DIN diet, TG1 2 ml/kg". Grid: animals with diet induced NASH which received 5 ml/kg TG1, indicated as "DIN diet, TG1 5 ml/kg. Diagonal lines: animals with diet induced NASH which received 2 ml/kg TG2, indicated as "DIN diet, TG2 2 ml/kg". Vertical lines: animals with diet induced NASH which received 5 ml/kg TG2, indicated as "DIN diet, TG1 5 ml/kg. Checkered bar: animals with diet induced NASH which received telmisartan mixed in the feed, indicated as "Telmisartan in DIN diet". *** indicates a statistically significant difference to the control chow + 0.9% saline group (p<0.001, Mann-Whitney test). # indicates a statistically significant difference to the DIN diet + 0.9% saline group (p<0.05, Mann-Whitney test). ## indicates a statistically significant difference to the DIN diet + 0.9% saline group (p<0.01, Mann-Whitney test).

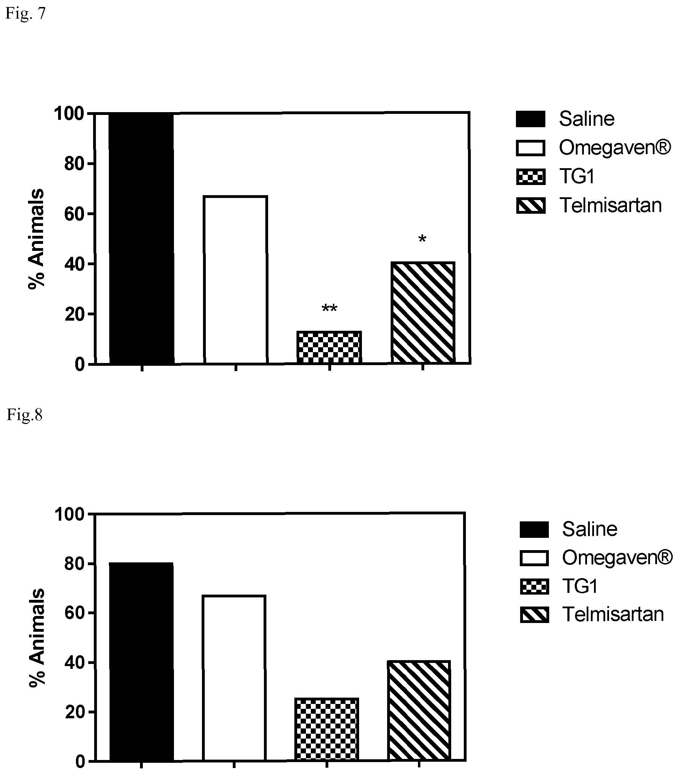

**Figure** 7 shows the incidence (in percent) of vacuolated foci of altered hepatocytes in mice with non-alcoholic steatohepatitis (NASH) progressing to hepatocellular carcinoma (HCC) as observed in Example 2. The respective treatments are indicated by the following symbols. Black bar: 5 ml/kg saline, indicated as "Saline". White bar: 5 ml/kg Omegaven^{®}, indicated as "Omegaven^{®}". Checkered bar: 5 ml/kg TG1, indicated as "TG1". Diagonal lines: 10 ml/kg telmisartan, indicated as "Telmisartan". * indicates a statistically significant difference to the saline group (p<0.05, chi-squared test). ** indicates a statistically significant difference to the saline group (p<0.01, chi-squared test).
**Figure** 8 shows the incidence (in percent) of adenomas in mice with non-alcoholic steatohepatitis (NASH) progressing to hepatocellular carcinoma (HCC) as observed in Example 2. The respective treatments are indicated by the following symbols. Black bar: 5 ml/kg saline, indicated as "Saline". White bar: 5 ml/kg Omegaven^{®}, indicated as "Omegaven^{®}". Checkered bar: 5 ml/kg TG1, indicated as "TG1". Diagonal lines: 10 ml/kg telmisartan, indicated as "Telmisartan".

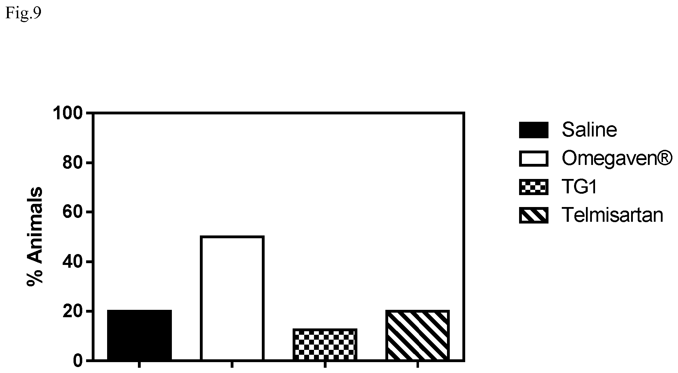

**Figure 9** shows the incidence (in percent) carcinomas in mice with non-alcoholic steatohepatitis (NASH) progressing to hepatocellular carcinoma (HCC) as observed in Example 2. The respective treatments are indicated by the following symbols. Black bar: 5 ml/kg saline, indicated as "Saline". White bar: 5 ml/kg Omegaven^{®}, indicated as "Omegaven^{®}". Checkered bar: 5 ml/kg TG1, indicated as "TG1". Diagonal lines: 10 ml/kg telmisartan, indicated as "Telmisartan".

### EXAMPLES

The following Examples shall merely illustrate the invention. They shall not be construed, whatsoever, to limit the scope of the invention.

### Example 1: NASH induced by high fat/cholesterol diet (DIN) can be treated by TG1

A diet induced NASH (DIN) model was used for investigating the effects of several EPA and DHA containing compositions. To this end, NASH was induced by using a 60% high fat/high cholesterol diet (Research Diets Inc., NJ, USA) and 10% fructose in drinking water (DIN model). Mice developed obesity and insulin resistance with concomitant liver complications over a period of 16 weeks, which clearly reflects the human metabolic syndrome.

The test items had the following compositions and were produced as follows:
Different mixtures comprising highly concentrated omega-3 fatty acids (eicosapentaenoic acid (EPA) and docosahexaenoic acid (DHA)) as triglycerides as obtained from Solutex S.L were used:
Mixture 1 (TG1) comprised 0.63 g/g EPA triglyceride and 0.14 g/g DHA triglyceride.
Mixture 2 (TG2) comprised 0.20 g/g EPA triglyceride and 0.46 g/g DHA triglyceride.

The TG1 and TG2 emulsions were prepared as follows: Using a shear mixer, lecithin (PL90, obtainable from egg yolk = egg lecithin with a phosphatidylcholine content of 64-79% and a phosphatidylethanolamine content of 10-18% by weight) was dispersed in water for injection, at a temperature between 55-60°C, previously containing glycerol and polyethylene glycol (PEG), using a Rayneri TURBOTEST high shear mixer, until a homogeneous dispersion was obtained. Afterwards, the pH of the aqueous dispersion was adjusted to 9-10. The oil phase, containing different ratios of docosahexaenoic acid/eicosapentaenoic acid triglycerides (mixtures 1 and 2), was heated to 55 °C and, then, oleic acid was added until a clear and homogeneous solution was obtained. The aqueous dispersion was then transferred to a separate container and the oil phase was added under continuous stirring, using a Rayneri TURBOTEST high shear mixer, to obtain coarse oil-in-water emulsions with oil phase concentrations comprised between 10-30 wt. The coarse emulsions were then passed six times through a homogenizer (Niro Soavi Panda Plus 2000) at 500 bar and a temperature between 50-60 °C. Finally, the emulsions were autoclaved at 122 °C for 15 min. Final lipid emulsions were obtained. The mean particle size of the lipid emulsions was measured using a Malvern Mastersizer 2000.

**Table 1: Composition of the emulsions**

| | |
|---|---|
| Ingredients: | Weight-% |
| Triglyceride - EPA/DHA | 10 (TG1: 0.63 g/g EPA to 0.14 g/g DHA; TG2: 0.20 g/g EPA to 0.46 g/g DHA) |
| Egg lecithin | 1.2 |
| Oleic acid | 0.15 |
| Glycerol | 2.25 |
| Polyethylene glycol PEG 400 | 1.0 |
| Water for injection | adds up to 100 |
| Properties: | |
| pH release | 8-8.7 |
| Surface mean droplet diameter D [3,2] | ≤ 0.3 µm |
| Volume weighted mean diameter D [4,3] | ≤ 0.3 µm |
| % Droplets > 5 µm | ≤ 0.05 |

Omegaven^{®} 10% was commercially available from Fresenius Kabi Deutschland GmbH.

Acute and chronic treatment effects were investigated directly after the treatment and 10 weeks after treatment start, respectively. Body weight was determined weekly. Blood glucose, plasma insulin, plasma alanine aminotransferase (ALT) and aspartate aminotransferase (AST) as well as total cholesterol and triglycerids were determined. After sacrifice of animals liver weight, hepatic cholesterol, and triglycerids were detected. Additionally liver histology was performed to investigate the non-alcoholic fatty liver disease activity scoring (NAS).

The study included the following groups:

| | |
|---|---|
| Group 1: | control chow and saline 0.9 % i.v., at 5 ml/kg n=15 |
| Group 2: | DIN diet and saline 0.9 % i.v., at 5 ml/kg n=15 |
| Group 3: | DIN diet and Omegaven^{®} 10% at 2 ml/kg, n=15 |
| Group 4: | DIN diet and Omegaven^{®} 10% at 5 ml/kg, n=15 |
| Group 5: | DIN diet and TG1 at 2 ml/kg, n=15 |
| Group 6: | DIN diet and TG1 at 5 ml/kg, n=15 |
| Group 7: | DIN diet and TG2 at 2 ml/kg, n=15 |
| Group 8: | DIN diet and TG2 at 5 ml/kg, n=15 |
| Group 9: | DIN diet and vehicle (saline 0.9%) i.v. at 5 ml/kg and telmisartan 5 mg/kg p.o. in the DIN diet, n=15 |

After a 1-week acclimatization period, animals received either normal diet and drinking water or high fat/high cholesterol diet and 10 % fructose in drinking water (DIN diet) for six weeks. After six weeks of diet treatment was started with a schedule of thrice i.v. administrations per week for a time period of 3 weeks. 2 and 5 ml/kg of test items were administered to the animals. Five animals per group were sacrificed after the three-week treatment period and the remaining animals were sacrificed 10 weeks after treatment initiation. Week 0 refers to the week of treatment initiation.

Continuous variables were graphically represented as mean + standard error of the mean (SEM). Differences between animals in group 1 (control chow and saline 0.9 % i.v. at 5 ml/kg) and group 2 (DIN diet and saline 0.9 % i.v. at 5 ml/kg) were evaluated using an unpaired t-test. Furthermore, treatment groups 3 to 8 and the positive control group (group 9) were statistically compared with group 2 using an unpaired t-test (except for the parameters body weight and body weight gain).

Results for the non-alcoholic fatty liver disease (NAFLD) activity scoring system (NAS) were graphically represented as mean ± SEM. Group 1 and group 2 were statistically compared with a Mann-Whitney test. Furthermore, treatment groups 3-8 and the positive control group 9 were compared against group 2 with a Mann-Whitney test.

Due to the explorative nature of the study, no correction for multiple comparisons was performed. All p-values are two-tailed and a p-value below 0.05 was considered statistically significant. The statistical analysis was performed with GraphPad Prism for Windows version 6.05.

No effects on the steatohepatitis were observed investigating the acute phase directly after treatment with both test items. However, beneficial effects were observed at ten weeks after treatment start with the low dose 2 ml/kg TG1. There was a reduction in the levels of liver injury parameters ALT (Figure 1) and AST (Figure 2) in the plasma. Additionally, a decrease in hepatic cholesterol (Figure 4), triglycerides fatty acids (Figure 5) and also the liver weight (Figure 3) was detected. The total NAS score which describes the extent of liver steatosis, ballooning of hepatocytes, liver inflammation and liver fibrosis was also reduced after TG1 treatment (Figure 6).

In contrast, Omegaven^{®} 10% did not exert any differences in the parameters mentioned above ten weeks after treatment start. TG1 contains high amounts of EPA and DHA in 10% oil. TG1 comprises EPA and DHA in a weight ratio between 6:1 and 4.5:1 ratios. The total amount of EPA and DHA ranges between 60-80 mg/ml. This quantity is a lot higher in comparison to the registered Fresenius Kabi product Omegaven^{®} 10% containing an EPA and DHA average of 50 mg/ml. Additionally the EPA and DHA ratio is approximately 1:1 in this product.

Another test item TG2 was also investigated in this model. TG2 is composed of a weight ratio between 1:2 and 1:4 EPA and DHA in 10% oil. However there were no beneficial treatment effects observed. In contrast, TG2 tended to aggravate liver steatosis as well as the severity of hepatic lesions at the highest dose leading to the assumption that the ratio of EPA and DHA is crucial for the treatment efficacy.

### Example 2: Effects of TG1 on liver cancer

In the second experiment Omegaven^{®} and TG1 were investigated in a murine NASH model progressing to HCC. Two days old male mice were injected subcutaneously with 20 µl of 10 mg/ml streptozotocin (STZ) solution (200 µg/mouse). Streptozotocin is a naturally occurring chemical that is particularly toxic to the insulin-producing beta cells of the pancreas in mammals. It is used in research to produce an animal model for diabetes. After 4 weeks of age, the mice were fed with a high fat diet ad libitum (57 kcal% fat, cat# HFD32, CLEA-Japan). Omegaven^{®} and TG1 were administered i.v. in a volume of 5 mL/kg thrice per week for duration of three weeks (6-9 weeks of age). Mice were kept until 20 weeks of age without any treatment until the end of the experiment. Individual body weight was measured daily during the treatment period and weekly after the end of treatment. All animals were sacrificed at 20 weeks of age. Livers were sampled and weighed as whole organs. The liver-to body weight ratio was calculated. Liver triglycerides were determined. The extirpated livers were macroscopically examined for the presence of gross swelling tumor nodules. Plasma ALT levels were investigated.

There were no beneficial treatment effects on liver weights, liver-to-body weight ratio, liver triglyceride and plasma ALT levels after treatment with both test items.

The liver sections were also examined histologically. In particular, H&E stained slides were made from liver tissue from the surviving animals in the experiment. The slides were examined by the study pathologist. The incidence of vacuolated foci of altered hepatocytes, adenomas and carcinomas in percent was graphically represented with bar diagrams (Figures 7 to 9). The difference between treatment groups and the control group was statistically evaluated using chi-squared test.

Histopathological examination of the liver sections revealed a positive treatment effect resulting in a reduced incidence of adenomas (Figure 10) and carcinomas (Figure 11) and reduced incidence and vacuolated foci of altered hepatocytes (Figure 9) after treatment with TG1 in comparison to the saline control group. The incidence and severity of the tumor lesions were comparable to the positive control telmisartan (group 4) in this experiment. Treatment with Omegaven^{®} resulted in no effect and incidence and severity of lesions comparable to the saline control.

Thus, short term administration of the test item TG1 surprisingly lead to improved outcome related to chronic effects in both models for liver disease.

### Cited references

Kalish BTl, Le HD, Gura KM, Bistrian BR, Puder M. A metabolomic analysis of two intravenous lipid emulsions in a murine model. PLoS One. 2013;8(4):e59653. doi: 10. 13711journal.pone.0059653.

Nogueira MA, Oliveira CP, Ferreira Alves VA, Stefano JT, Rodrigues LS, Torrinhas RS, Cogliati B, Barbeiro H, Carrilho FJ, Waitzberg DL. Omega-3 polyunsaturated fatty acids in treating non-alcoholic steatohepatitis: A randomized, double-blind, placebo-controlled trial. Clin Nutr. 2016 Jun;35(3):578-86. doi: 10.1016/j.clnu.2015.05.001.

Li YH, Yang LH, Sha KH, Liu TG, Zhang LG, Liu XX. Efficacy of poly-unsaturated fatty acid therapy on patients with nonalcoholic steatohepatitis. World J Gastroenterol. 2015 Jun 14;21(22):7008-13.

WO2015/113987.

WO2016/057915.

US2013/015861.

WO2015/053379.

S. Cazanave, B. Banini, A. Asgharpour, R. Vincent, M. Seneshaw, D. Prasssana Kumar, F. Mirshahi, K. Daita, P. Puri, J. Oscarsson, P. Bedossa, A. Sanyal. Omega-3 carboxylic acids, Epanova™, and the sodium-glucose co-transporter 2 inhibitor, Dapagliflozin™, improve steatohepatitis and fibrosis scoring in a mouse model of non-alcoholic steatohepatitis. Journal of Hepatology 2016; 64: S631-S832

## Claims

1. A composition for use in treating and/or preventing a liver disease in a subject, wherein said composition comprises eicosapentaenoic acid (EPA) and docosahexaenoic acid (DHA), preferably in esterified form, e.g., in triglyceride form, wherein said composition is administered to the subject parenterally and wherein said composition comprises eicosapentaenoic acid (EPA) and docosahexaenoic acid (DHA) in a weight ratio of between 2.0 (EPA) to 1.0 (DHA) and 10.0 (EPA) to 1.0 (DHA).

2. The composition for use according to claim 1, wherein said composition comprises an aqueous phase and an oil phase.

3. The composition for use according to claim 2, wherein the oil phase of said composition amounts to at least 30% by weight based on the total weight of the composition.

4. The composition for use according to any one of claims 1 to 3, wherein said composition further comprises at least one amphoteric surfactant, at least one co-surfactant and at least one co-solvent.

5. Composition for use of any one according to claims 1 to 4 wherein the daily dosage of EPA and DHA is from 400 µg to 400 mg per kg body weight, preferably from 1 mg to 200 mg, especially in the range of 1 mg to 100 mg and most preferred from 1 mg to 60 mg.

6. Composition for use of any one per kg body weight claims 1 to 5 wherein the daily dosage of EPA and DHA is below 100 mg per kg body weight.

7. The composition for use according to any one of claims 1 to 6, wherein said composition is administered within of four weeks after onset or diagnosis of said liver disease.

8. The composition for use according to any one of claims 1 to 7, wherein said composition is first administered within three weeks after onset or diagnosis of said liver disease.

9. The composition for use according to any one of claims 1 to 7, wherein said composition is administered one to three times per week.

10. The composition for use according to any one of claims 1 to 9, wherein said liver disease is selected from the group consisting of: steatohepatitis, fatty liver, liver steatosis, liver inflammation, liver fibrosis, liver cirrhosis and liver cancer.

11. The composition for use according to any one of claims 1 to 9, wherein said subject is suffering from non-alcoholic fatty liver or NASH.

## Patentansprüche

1. Zusammensetzung zur Verwendung bei der Behandlung und/oder Vorbeugung einer Lebererkrankung bei einem Subjekt, wobei die Zusammensetzung Eicosapentaensäure (EPA) und Docosahexaensäure (DHA), vorzugsweise in veresterter Form, z. B. in Triglyceridform, umfasst, wobei die Zusammensetzung dem Subjekt parenteral verabreicht wird und wobei die Zusammensetzung Eicosapentaensäure (EPA) und Docosahexaensäure (DHA) in einem Gewichtsverhältnis zwischen 2,0 (EPA) zu 1,0 (DHA) und 10,0 (EPA) zu 1,0 (DHA) umfasst.

2. Zusammensetzung zur Verwendung nach Anspruch 1, wobei die Zusammensetzung eine wässrige Phase und eine Ölphase umfasst.

3. Zusammensetzung zur Verwendung nach Anspruch 2, wobei die Ölphase der Zusammensetzung mindestens 30 Gew.-%, bezogen auf das Gesamtgewicht der Zusammensetzung, ausmacht.

4. Zusammensetzung zur Verwendung nach einem der Ansprüche 1 bis 3, wobei die Zusammensetzung ferner mindestens ein amphoteres Tensid, mindestens ein Co-Tensid und mindestens ein Co-Lösemittel umfasst.

5. Zusammensetzung zur Verwendung nach einem der Ansprüche 1 bis 4, wobei die tägliche Dosierung von EPA und DHA 400 µg bis 400 mg pro kg Körpergewicht, vorzugsweise 1 mg bis 200 mg, insbesondere im Bereich von 1 mg bis 100 mg und am bevorzugtesten 1 mg bis 60 mg beträgt.

6. Zusammensetzung zur Verwendung nach einem der Ansprüche 1 bis 5, wobei die tägliche Dosierung von EPA und DHA unter 100 mg pro kg Körpergewicht liegt.

7. Zusammensetzung zur Verwendung nach einem der Ansprüche 1 bis 6, wobei die Zusammensetzung innerhalb von vier Wochen nach Beginn oder Diagnose der Lebererkrankung verabreicht wird.

8. Zusammensetzung zur Verwendung nach einem der Ansprüche 1 bis 7, wobei die Zusammensetzung erstmals innerhalb von drei Wochen nach Beginn oder Diagnose der Lebererkrankung verabreicht wird.

9. Zusammensetzung zur Verwendung nach einem der Ansprüche 1 bis 7, wobei die Zusammensetzung ein- bis dreimal pro Woche verabreicht wird.

10. Zusammensetzung zur Verwendung nach einem der Ansprüche 1 bis 9, wobei die Lebererkrankung aus der Gruppe ausgewählt ist, die aus: Steatohepatitis, Fettleber, Lebersteatose, Leberentzündung, Leberfibrose, Leberzirrhose und Leberkrebs besteht.

11. Zusammensetzung zur Verwendung nach einem der Ansprüche 1 bis 9, wobei das Subjekt an einer nichtalkoholischen Fettleber oder NASH leidet.

## Revendications

1. Composition destinée à être utilisée dans le traitement et/ou la prévention d'une maladie du foie chez un sujet, dans laquelle ladite composition comprend de l'acide eicosapentaénoïque (EPA) et de l'acide docosahexaénoïque (DHA), de préférence sous forme estérifiée, par exemple, sous forme de triglycéride, dans laquelle ladite composition est administrée au sujet par voie parentérale et dans laquelle ladite composition comprend de l'acide eicosapentaénoïque (EPA) et de l'acide docosahexaénoïque (DHA) dans un rapport en poids entre 2,0 (EPA) et 1,0 (DHA) et 10,0 (EPA) et 1,0 (DHA).

2. Composition destinée à être utilisée selon la revendication 1, dans laquelle ladite composition comprend une phase aqueuse et une phase huileuse.

3. Composition destinée à être utilisée selon la revendication 2, dans laquelle la phase huileuse de ladite composition représente au moins 30 % en poids par rapport au poids total de la composition.

4. Composition destinée à être utilisée selon l'une quelconque des revendications 1 à 3, dans laquelle ladite composition comprend en outre au moins un tensioactif amphotère, au moins un co-tensioactif et au moins un cosolvant.

5. Composition destinée à être utilisée selon l'une quelconque des revendications 1 à 4 dans laquelle la dose quotidienne d'EPA et de DHA va de 400 µg à 400 mg par kg de poids corporel, de préférence de 1 mg à 200 mg, en particulier dans la plage de 1 mg à 100 mg et le plus préférablement de 1 mg à 60 mg.

6. Composition destinée à être utilisée selon l'une quelconque des revendications 1 à 5 dans laquelle la dose quotidienne d'EPA et de DHA est en dessous de 100 mg par kg de poids corporel.

7. Composition destinée à être utilisée selon l'une quelconque des revendications 1 à 6, dans laquelle ladite composition est administrée dans les quatre semaines suivant l'apparition ou le diagnostic de ladite maladie du foie.

8. Composition destinée à être utilisée selon l'une quelconque des revendications 1 à 7, dans laquelle ladite composition est d'abord administrée dans les trois semaines suivant l'apparition ou le diagnostic de ladite maladie du foie.

9. Composition destinée à être utilisée selon l'une quelconque des revendications 1 à 7, dans laquelle ladite composition est administrée une à trois fois par semaine.

10. Composition destinée à être utilisée selon l'une quelconque des revendications 1 à 9, dans laquelle ladite maladie du foie est choisie dans le groupe constitué de : stéatohépatite, stéatose hépatique, maladie du foie gras, inflammation du foie, fibrose du foie, cirrhose du foie et cancer du foie.

11. Composition destinée à être utilisée selon l'une quelconque des revendications 1 à 9, dans laquelle ledit sujet souffre d'une stéatose hépatique non alcoolique ou NASH.
